# EUROPÄISCHE PATENTSCHRIFT

(11) **EP 1 313 724 B1**
(45) Veröffentlichungstag und Bekanntmachung des Hinweises auf die Patenterteilung: **28.04.2004**
(21) Anmeldenummer: 01925442.4
(22) Anmeldetag: 14.03.2001
(51) Int. Cl.: C07D 333/20, C07D 307/52, C07D 233/54, C07D 277/28, A61K 7/13

(54) **N-HETEROARYLMETHYL-P-PHENYLENDIAMIN-DERIVATE SOWIE DIESE VERBINDUNGEN ENTHALTENDE OXIDATIONSFÄRBEMITTEL**
N-HETEROARYLMETHYL-P-PHENYLENDIAMINE DERIVATIVES AND COMPOUNDS CONTAINING OXIDATION COLOURING AGENTS
DERIVES N-HETEROARYLMETHYL-P-PHENYLENDIAMINE ET COLORANTS CAPILLAIRES PAR OXYDATION CONTENANT CES COMPOSES

(30) Priorität: 31.08.2000 DE 10042786
(43) Veröffentlichungstag der Anmeldung: 28.05.2003
(73) Patentinhaber: Wella Aktiengesellschaft, 64274 Darmstadt (DE)
(72) Erfinder: CHASSOT, Laurent, CH-1724 Praroman (CH); BRAUN, Hans-Jürgen, CH-3182 Überstorf (CH)
(86) Internationale Anmeldenummer: PCT/EP2001/002840
(87) Internationale Veröffentlichungsnummer: WO 2002/018365

(56) Entgegenhaltungen:
- EP-A- 0 984 007
- DE-A- 4 110 995
- US-A- 5 183 941
- US-A- 5 540 738
- US-A- 6 042 620

## Beschreibung

Die Erfindung betrifft neue N-Heteroarylmethyl-p-phenylendiamin-Derivate sowie diese Verbindungen enthaltende Mittel zum oxidativen Färben von Keratinfasem.

Auf dem Gebiet der Färbung von Keratinfasem, insbesondere der Haarfärbung, haben Oxidationsfarbstoffe eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Als Entwicklersubstanzen werden hierbei insbesondere 2,5-Diaminotoluol, 2,5-Diaminophenylethylalkohol, p-Aminophenol und 1,4-Diaminobenzol eingesetzt, während als Kupplersubstanzen beispielsweise Resorcin, 4-Chlorresorcin, 1-Naphthol, 3-Aminophenol und Derivate des m-Phenylendiamins zu nennen sind.
An Oxidationsfarbstoffe, die zur Färbung menschlicher Haare verwendet werden, werden neben der Färbung in der gewünschten Intensität zahlreiche zusätzliche Anforderungen gestellt. So müssen die Farbstoffe in toxikologischer und dermatologischer Hinsicht unbedenklich sein und die erzielten Haarfärbungen eine gute Lichtechtheit, Dauerwellechtheit, Säureechtheit und Reibeechtheit aufweisen. Auf jeden Fall aber müssen solche Färbungen ohne Einwirkung von Licht, Reibung und chemischen Mitteln über einen Zeitraum von mindestens 4 bis 6 Wochen stabil bleiben. Außerdem ist es erforderlich, daß durch Kombination geeigneter Entwicklersubstanzen und Kupplersubstanzen eine breite Palette verschiedener Farbnuancen erzeugt werden kann.

In der US 5 540 738 werden Oxidationshaarfärbemittel beschrieben, welche u.a. N-Furfuryl-p-phenylendiamin sowie ein Jodid enthalten. Diese Färbemittel sind jedoch nicht in jeder Hinsicht befriedigend, insbesondere kann ohne den Zusatz von Jodid kein befriedigendes Färbeergebnis erzielt werden.

Es bestand daher weiterhin ein dringendes Bedürfnis für neue Farbstoffvorstufen für Oxidationsfärbemittel, welche auch ohne den Zusatz von Jodid intensive Färbungen von Keratinfasern ermöglichen.

Es wurde nun gefunden, dass intensivere braune, blaue und rote Farbnuancen erhalten werden, wenn man N-Heteroarylmethyl-p-phenylendiamin-Derivate gemäß der allgemeinen Formel (I) zusammen mit üblichen Kupplerverbindungen zur Kupplung bringt.

Gegenstand der vorliegende Erfindung sind daher N-Heteroarylmethyl-p-phenylendiamin-Derivate der allgemeinen Formel (I) oder deren physiologisch verträgliche, wasserlösliche Salze, 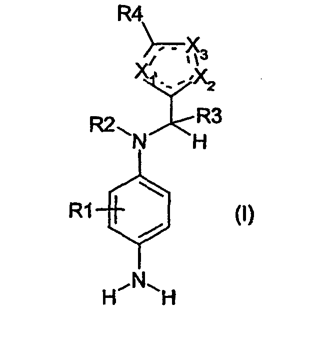
- **X1**: gleich Schwefel, Stickstoff, C-R6 oder N-R5 ist;
- **X2**: gleich Schwefel, Stickstoff, C-R6 oder N-R5 ist
- **X3**: gleich Schwefel, Stickstoff, Sauerstoff, C-R6 oder N-R5 ist;
- **R1**: gleich Wasserstoff, einer C₁-C₄-Alkylgruppe oder einer C₁-C₄-Hydroxyalkylgruppe ist;
- **R2,R3**: gleich oder verschieden sein können und unabhängig voneinander gleich Wasserstoff oder einer C₁-C₆-Alkylgruppe sind;
- **R4,R6**: gleich oder verschieden sein können und unabhängig voneinander gleich Wasserstoff, einem Halogenatom, einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer C₁-C₄-Alkylaminogruppe, einer Di(C₁-C₄)-alkylaminogruppe, einer Di(C₁-C₄-hydroxy-alkyl)aminogruppe, einer C₁-C₄-Hydroxyalkyl-amino-gruppe, einer Trifluormethangruppe, einer -C(O)CH₃-Gruppe, einer -C(O)CF₃-Gruppe, einer -Si(CH₃)₃-Gruppe, einer C₁-C₄-Hydroxyalkylgruppe oder einer C₃-C₄ Dihydroxyalkylgruppe sind;
- **R5**: gleich Wasserstoff, einer C₁-C₆-Alkylgruppe, einer C₁-C₄-Hydroxyalkylgruppe, einer Phenylgruppe oder einer Acetylgruppe ist;
wobei mindestens einer und maximal zwei der Reste **X1** bis **X3** gleich C-R6 sind und maximal einer der Reste **X1** bis **X3** gleich Schwefel, Sauerstoff oder N-R5 ist.

Als erfindungsgemäß geeigneteVerbindungen der Formel (I) können beispielweise genannt werden:
N-Thiophen-3-ylmethyl-1,4-diamino-benzol, N-Furan-3-ylmethyl-1,4-diamino-benzol, N-(1 H-Imidazol-2-ylmethyl)-1,4-diamino-benzol, N-(1 H-Pyrol-2-ylmethyl)-1,4-diamino-benzol, N-(N-Methyl-pyrol-2-ylmethyl)-1,4-diamino-benzol, N-Thiophen-2-ylmethyl-1,4-diamino-benzol, N-Thiazol-2-ylmethyl-1,4-diamino-benzol, N-(5-Nitro-thiophen-2-ylmethyl)-1,4-diamino-benzol, N-(3-Methyl-thiophen-2-ylmethyl)-1,4-diamino-benzol, N-(2-Methyl-thiophen-3-ylmethyl)-1,4-diamino-benzol, N-(4-Methylthiophen-3-ylmethyl)-1 ,4-diamino-benzol, N-(5-Methyl-thiophen-2-ylmethyl)-1,4-diamino-benzol, N-(3-Chlor-thiophen-2-ylmethyl)-1,4-diamino-benzol, N-(4-Methyl-thiophen-2-ylmethyl)-1,4-diamino-benzol, N-(4-Chlor-thiophen-2-ylmethyl)-1,4-diamino-benzol, N-(5-Methylthiophen-2-ylmethyl)-1 ,4-diamino-benzol, N-(5-Chlor-thiophen-2-ylmethyl)-1,4-diamino-benzol, 2-[(4-Amino-phenyl)-thiophen-2-ylmethyl-amino]-ethanol, 2-[(4-Amino-phenyl)- 1 H-Imidazol -2-ylmethyl-amino]-ethanol, N-Methyl-N-thiophen-3-ylmethyl-1,4-diamino-benzol, N-Methyl-N-furan-3-ylmethyl-1,4-diamino-benzol, N-Methyl-N-(1H-imidazol-2-ylmethyl)-1,4-diamino-benzol, N1-(1H-Imidazol-2-ylmethyl)-1,4-diamino-2-methylbenzol, N4-(1H-Imidazol-2-ylmethyl)-1,4-diamino-2-methyl-benzol, N1-Furan-3-ylmethyl-1,4-diamino-2-methyl-benzol, N4-Furan-3-ylmethyl-1,4-diamino-2-methyl-benzol, N1-Thiophen-2-ylmethyl-1,4-diamino-2-methyl-benzol, N4-thiophen-2-ylmethyl-1,4-diamino-2-methyl- benzol, 2-{5-Amino-2-[(furan-2-ylmethyl)-amino]-phenyl}-ethanol, 2-{6-Amino-3-[(furan-2-ylmethyl)-amino]-phenyl}-ethanol, 2-{5-Amino-2-[{thiophen-3-ylmethyl)-amino]-phenyl}-ethanol, 2-{6-Amino-3-[(thiophen-3-ylmethyl)-amino]-phenyl}-ethanol 2-{5-Amino-2-[(thiophen-2-ylmethyl)-amino]-phenyl}-ethanol, 2-{6-Amino-3-[(thiophen-2-ylmethyl)-amino]-phenyl}-ethanol;

Bevorzugt sind Verbindungen der Formel (I) in denen (i) **R1, R2** und **R3** oder **R1, R2, R3** und **R4** Wasserstoff bedeuten oder (ii) **R1, R2** und **R3** oder **R1, R2, R3** und **R4** Wasserstoff bedeuten und **X1** gleich Schwefel oder N-H ist und **X2** gleich Stickstoff oder C-R6 ist und **X3** gleich C-R6 ist oder (iii) **R1, R2** und **R3** oder **R1, R2, R3** und **R4** gleich Wasserstoff sind und **X3** gleich Schwefel oder Sauerstoff ist und **X1** und **X2** gleich C-R6 sind oder (iv) **R1, R2** und **R3** oder **R1, R2, R3** und **R4** gleich Wasserstoff sind und **X2** gleich Schwefel oder Sauerstoff ist und **X1** und **X3** gleich C-R6 sind.

Im Sinne der Gesamterfindung besonders geeignete N-Heteroarylmethyl-p-phenylendiamin-Derivate der Formel (I) sind N-Thiophen-3-ylmethyl-1,4-diamino-benzol, N-Furan-3-ylmethyl-1,4-diamino-benzol, N-(1H-Imidazol-2-ylmethyl)-1,4-diamino-benzol, N-(1H-Pyrrol-2-ylmethyl)-1,4-diaminobenzol und N-Thiophen-2-ylmethyl-1,4-diamino-benzol oder deren physiologisch verträgliche Salze.

Die Verbindungen der Formel (I) können sowohl als freie Basen als auch in Form ihrer physiologisch verträglichen Salze mit anorganischen oder organischen Säuren, wie zum Beispiel Salzsäure, Schwefelsäure, Phosphorsäure, Essigsäure, Propionsäure, Milchsäure oder Zitronensäure, eingesetzt werden.

Die Herstellung der erfindungsgemäßen N-Heteroarylmethyl-p-phenylendiamin-Derivate der Formel (I) kann unter Verwendung von bekannten Syntheseverfahren erfolgen. Die Synthese der Verbindungen der Formel (I) kann beispielsweise wie folgt durchgeführt werden:
(1) Durch eine reduktive Alkylierung eines substituierten Benzols der Formel (II) 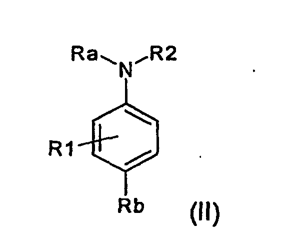 mit einer Heteroarylverbindung der Formel (III) 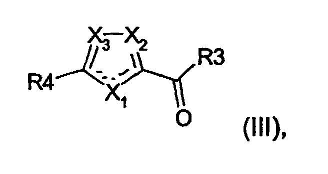 worin
   **Rb** die Bedeutung NHRa oder NO₂ hat,
   **Ra** eine Schutzgruppe darstellt, wie sie zum Beispiel in dem Kapitel "Protective Groups" in Organic Synthesis, Kapitel 7, Wiley Interscience, 1991 beschrieben wird,
   **X1, X2, X3, R1, R2, R3** und **R4** die in Formel (I) gennante Bedeutung haben, und
(2) Abspaltung der Schutzgruppe oder Abspaltung der Schutzgruppe und Reduktion der Nitrogruppe.

Die Verbindungen der Formel (I) ermöglichen eine breite Palette verschiedener Farbtöne, die sich von blonden über braune, purpume, violette bis hin zu blauen und schwarzen Farbtönen erstreckt und eignen sich hervorragend für die Verwendung in Färbemitteln für Keratinfasern.

Ein weiterer Gegenstand der vorliegenden Erfindung sind daher Mittel zum oxidativen Färben von Keratinfasern, wie zum Beispiel Haaren, Pelzen, Federn oder Wolle, und insbesondere menschlichen Haaren, auf der Basis einer Entwicklersubstanz-Kupplersubstanz-Kombination, welche als Entwicklersubstanz mindestens ein N-Heteroarylmethyl-p-phenylendiamin-Derivat der vorstehend beschriebenen Formel (I) enthalten.

Das N-Heteroarylmethyl-p-phenylendiamin-Derivat der Formel (I) wird in dem erfindungsgemäßen Färbemittel vorzugsweise in einer Menge von etwa 0,005 bis 20 Gewichtsprozent verwendet, wobei eine Einsatzmenge von etwa 0,01 bis 5,0 Gewichtsprozent und insbesondere 0,1 bis 2,5 Gewichtsprozent besonders bevorzugt ist.

Als Kupplersubstanzen kommen vorzugsweise 2,6-Diamino-pyridin, 2-Amino-4-[(2-hydroxyethyl)amino]-anisol, 2,4-Diamino-1-fluor-5-methylbenzol, 2,4-Diamino-1-methoxy-5-methyl-benzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylamino-benzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl}-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methylphenol, 5-Amino-4-methoxy-2-methyl-phenol, 5-Amino-4-ethoxy-2-methylphenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methylphenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphtholacetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion in Betracht.

Obwohl die vorteilhaften Eigenschaften der hier beschriebenen p-Diaminobenzol-Derivate der Formel (I) es nahelegen, diese als alleinige Entwicklersubstanz zu verwenden, ist es selbstverständlich auch möglich, die p-Diaminobenzol-Derivate der Formel (I) gemeinsam mit bekannten Entwicklersubstanzen, wie zum Beispiel 1,4-Diaminobenzol, 2,5-Diaminotoluol, 2,5-Diaminophenylethylalkohol, 4-Aminophenol und seinen Derivaten, beispielsweise 4-Amino-3-methylphenol, 4,5-Diamino-1-(2-hydroxyethyl)-pyrazol oder Tetraaminopyrimidinen, einzusetzen.

Die Kupplersubstanzen und Entwicklersubstanzen können in dem erfindungsgemäßen Färbemittel jeweils einzeln oder im Gemisch miteinander enthalten sein, wobei die Gesamtmenge an Kupplersubstanzen und Entwicklersubstanzen in dem erfindungsgemäßen Färbemittel (bezogen auf die Gesamtmenge des Färbemittels) jeweils etwa 0,005 bis 20 Gewichtsprozent, vorzugsweise etwa 0,01 bis 5,0 Gewichtsprozent und insbesondere 0,1 bis 2,5 Gewichtsprozent, beträgt.

Die Gesamtmenge der in dem hier beschriebenen Färbemittel enthaltenen Entwicklersubstanz-Kupplersubstanz-Kombination beträgt vorzugsweise etwa 0,01 bis 20 Gewichtsprozent, wobei eine Menge von etwa 0,02 bis 10 Gewichtsprozent und insbesondere 0,2 bis 6 Gewichtsprozent besonders bevorzugt ist. Die Entwicklersubstanzen und Kupplersubstanzen werden im allgemeinen in etwa äquimolaren Mengen eingesetzt; es ist jedoch nicht nachteilig, wenn die Entwicklersubstanzen diesbezüglich in einem gewissen Uberschuß oder Unterschuß (beispielsweise in einem Verhältnis (Kuppler : Entwickler) von 1:2 bis 1:0,5) vorhanden sind.

Weiterhin kann das erfindungsgemäße Färbemittel zusätzlich andere Farbkomponenten, beispielsweise 6-Amino-2-methylphenol und 2-Amino-5-methylphenol, sowie ferner übliche direktziehende Farbstoffe, zum Beispiel Triphenylmethanfarbstoffe wie 4-[(4'-aminophenyl)-(4'-imino-2",5"cyclohexadien-1"-yliden)-methyl]-2-methylaminobenzol-monohydrochlorid (C.I. 42 510) und 4-[(4'-amino-3'-methyl-phenyl)-(4"-imino-3"-methyl-2",5"-cyclohexadien-1"-yliden)-methyl]-2-methyl-aminobenzol monohydrochlorid (C.I. 42 520), aromatische Nitrofarbstoffe wie 4-(2'-hydroxyethyl)aminonitrotoluol, 2-Amino-4,6-dinitrophenol, 2-Amino-5-(2'-hydroxyethyl)aminonitrobenzol, 2-Chlor-6-(ethylamino)-4-nitrophenol, 4-Chlor-N-(2-hydroxyethyl-2-nitroanilin, 5-Chlor-2-hydroxy-4-nitroanilin, 2-Amino-4-chlor-6-nitrophenol und 1-[(2'-Ureidoethyl)amino-4-nitrobenzol, Azofarbstoffe wie 6-[(4'-Aminophenyl)azo]-5-hydroxy-naphthalin-1-sulfonsäure-Natriumsalz (C.I. 14 805) und Dispersionsfarbstoffe wie beispielsweise 1,4-Diaminoanthrachinon und 1,4,5,8-Tetraaminoantrachinon, enthalten. Die Färbemittel können diese Farbkomponenten in einer Menge von etwa 0,1 bis 4 Gewichtsprozent enthalten.

Selbstverständlich können die Kupplersubstanzen und Entwicklersubstanzen sowie die anderen Farbkomponenten, sofern es Basen sind, auch in Form der physiologisch verträglichen Salze mit organischen oder anorganischen Säuren, wie beispielsweise Salzsäure oder Schwefelsäure, beziehungsweise - sofern sie aromatische OH-Gruppen besitzen - in Form der Salze mit Basen, zum Beispiel als Alkaliphenolate, eingesetzt werden.

Darüber hinaus können in den Färbemitteln, falls diese zur Färbung von Haaren verwendet werden sollen, noch weitere übliche kosmetische Zusätze, beispielsweise Antioxidantien wie Ascorbinsäure, Thioglykolsäure oder Natriumsulfit, sowie Parfümöle, Komplexbildner, Netzmittel, Emulgatoren, Verdicker und Pflegestoffe enthalten sein.
Die Zubereitungsform des erfindungsgemäßen Färbemittels kann beispielsweise eine Lösung, insbesondere eine wäßrige oder wäßrigalkoholische Lösung sein. Die besonders bevorzugten Zubereitungsformen sind jedoch eine Creme, ein Gel oder eine Emulsion. Ihre Zusammensetzung stellt eine Mischung der Farbstoffkomponenten mit den für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin oder Glykole wie 1,2-Propylenglykol, weiterhin Netzmittel oder Emulgatoren aus den Klassen der anionischen, kationischen, amphoteren oder nichtionogenen oberflächenaktiven Substanzen wie zum Beispiel Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide und oxethylierte Fettsäureester ferner Verdicker wie hohere Fettalkohole, Stärke, Cellulosederivate, Petrolatum, Paraffinöl und Fettsäuren, sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Je nach Zusammensetzung kann das erfindungsgemäße Färbemittel schwach sauer, neutral oder alkalisch reagieren. Insbesondere weist es einen pH-Wert von 6,5 bis 11,5 auf, wobei die basische Einstellung vorzugsweise mit Ammoniak erfolgt. Es können aber auch organische Amine, zum Beispiel Monoethanolamin und Triethanolamin, oder auch anorganische Basen wie Natriumhydroxid und Kaliumhydroxid Verwendung finden. Für eine pH-Einstellung im sauren Bereich kommen anorganische oder organische Säuren, zum Beispiel Phosphorsäure, Essigsäure Zitronensäure oder Weinsäure, in Betracht.

Für die Anwendung zur oxidativen Färbung von Haaren vermischt man das vorstehend beschriebene Färbemittel unmittelbar vor dem Gebrauch mit einem Oxidationsmittel und trägt eine für die Haarfärbebehandlung ausreichende Menge, je nach Haarfülle, im allgemeinen etwa 60 bis 200 Gramm, dieses Gemisches auf das Haar auf.

Als Oxidationsmittel zur Entwicklung der Haarfärbung kommen hauptsächlich Wasserstoffperoxid oder dessen Additionsverbindungen an Harnstoff, Melamin, Natriumborat oder Natriumcarbonat in Form einer 3-bis 12-prozentigen, vorzugsweise 6-prozentigen, wässrigen Lösung, aber auch Luftsauerstoff in Betracht. Wird eine 6-prozentige Wasserstoffperoxid-Lösung als Oxidationsmittel verwendet, so beträgt das Gewichtsverhältnis zwischen Haarfärbemittel und Oxidationsmittel 5:1 bis 1:2, vorzugeweise jedoch 1:1. Größere Mengen an Oxidationsmittel werden vor allem bei höheren Farbstoffkonzentrationen im Haarfärbemittel, oder wenn gleichzeitig eine stärkere Bleichung des Haares beabsichtigt ist, verwendet. Man läßt das Gemisch bei 15 bis 50 Grad Celsius etwa 10 bis 45 Minuten lang, vorzugsweise 30 Minuten lang, auf das Haar einwirken, spült sodann das Haar mit Wasser aus und trocknet es. Gegebenenfalls wird im Anschluß an diese Spülung mit einem Shampoo gewaschen und eventuell mit einer schwachen organischen Säure, wie zum Beispiel Zitronensäure oder Weinsäure, nachgespült. Anschließend wird das Haar getrocknet.

Die erfindungsgemäßen Färbemittel mit einem Gehalt an Diaminobenzol-Derivaten der Formel (I) als Entwicklersubstanz ermöglichen Färbungen mit ausgezeichneter Farbechtheit, insbesondere was die Lichtechtheit, Waschechtheit und Reibeechtheit anbetrifft. Hinsichtlich der färberischen Eigenschaften bieten die erfindungsgemäßen Färbemittel je nach Art und Zusammensetzung der Farbkomponenten eine breite Palette verschiedener Farbnuancen, welche sich von blonden über braune, purpurne, violette bis hin zu blauen und schwarzen Farbtönen erstreckt. Hierbei zeichnen sich die Farbtöne durch ihre besondere Farbintensität aus. Die sehr guten färberischen Eigenschaften der Färbemittel gemäß der vorliegenden Anmeldung zeigen sich weiterhin darin, daß diese Mittel insbesondere auch eine Anfärbung von ergrauten, chemisch nicht vorgeschädigten Haaren problemlos und mit guter Deckkraft ermöglichen.

Die in dem erfindungsgemäßen Mittel verwendeten N-Heteroarylmethyl-p-phenylendiamin-Derivate der Formel (I) sind gut in Wasser löslich und ermöglichen Färbungen mit hoher Farbintensität und ausgezeichneter Farbechtheit, insbesondere was die Lichtechtheit, Waschechtheit und Reibeechtheit anbetrifft. Sie weisen weiterhin eine ausgezeichnete Lagerstabilität, insbesondere als Bestandteil der vorstehend beschriebenen Färbemittel, auf.

Die nachfolgenden Beispiele sollen den Gegenstand der Erfindung näher erläutern, ohne ihn darauf zu beschränken.

### Beispiele

**Beispiele 1:** Synthese von N-Heteroarylmethyl-p-phenylendiaminen 0,031 g (0,15 mmol) N-(4-Aminophenyl)-carbaminsäure-tert-butylester und 0,1 mmol des entsprechenden Aldehyds werden in 1,2-Dichlorethan gelöst. Anschliessend werden 0,1 ml einer Essigsäurelösung (1-molar in 1,2-Dichlorethan) und 0,06 g NaBH(OAc)₃ (0,3 mmol) zugegeben und die Reaktionmischung wird 5 bis 15 Stunden bei Raumtemperatur gerührt. Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol und 1,5 ml einer 2,9 molaren ethanolische Salzsäurelösung auf 50 °C erwärmt. Der Niederschlag wird abfiltriert, zweimal mit 1 ml Ethanol gewaschen und sodann getrocknet.
**a.** N-Thiophen-3-ylmethyl-1,4-diamino-benzol Hydrochlorid
   Verwendeter Aldehyd: Thiophen-3-carbaldehyd
   Ausbeute: 0,025g (90% der Theorie)
   Massenspketrum: MH+ 205(100)
**b.** N-Furan-3-ylmethyl-1,4-diamino-benzol Hydrochlorid
   Verwendeter Aldehyd: Furan-3-carbaldehyd
   Ausbeute: 0,025g (95% der Theorie)
   Massenspketrum: MH+ 189(100)
**c.** N-(1H-Imidazol-2-ylmethyl)-1,4-diamino-benzol Hydrochlorid
   Verwendeter Aldehyd: Imidazol-2-carbaldehyd
   Ausbeute: 0,025g (83% der Theorie)
   Massenspketrum: MH+ 189(100)
d. N-Thiophen-2-ylmethyl-1,4-diamino-benzol Hydrochlorid
   Verwendete Aldehydderivat: Thiophen-2-carbaldehyd
   Ausbeute: 0,025g (90% der Theorie)
   Massenspketrum: MH+ 205(100)
**e.** N-Thiazol-2-ylmethyl-1,4-diamino-benzol Hydrochlorid
   Verwendete Aldehydderivat: Thiazol-2-carbaldehyd
   Ausbeute: 0,025g (79% der Theorie)
   Massenspketrum: MH+ 316(100)
**f.** N-(5-Nitro-thiophen-2-ylmethyl)-1,4-diamino-benzol Hydrochlorid
   Verwendete Aldehydderivat: 5-Nitro-thiophen-2-carbaldehyd
   Ausbeute: 0,025g (77% der Theorie)
   Massenspketrum: MH+ 250(60)

**Beispiele 2:** Synthese von N1-Heteroarylmethyl-2-methyl-1,4-diaminobenzolen und N4-Heteroarylmethyl-2-methyl-1,4-diaminobenzolen 0,033 g (0,15 mmol) einer Mischung aus N-(4-Amino-2-methyl-phenyl)-carbaminsäure-tert-butylester und N-(4-Amino-3-methyl-phenyl)-carbaminsäure-tert-butylester und 0,10 mmol des entsprechenden Aldehyds werden in 1,2-Dichlorethan gelöst. Anschliessend werden 0,1 ml einer Essigsäurelösung (1-molar in 1,2-Dichlorethan) und 0,06 g NaBH(OAC)₃ (0,3 mmol) zugegeben und die Reaktionmischung wird 5 bis 15 Stunden bei Raumtemperatur gerührt. Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol und 1,5 ml einer 2,9-molaren ethanolische Salzsäurelösung auf 50 °C erwärmt.
Der Niederschlag wird abfiltriert, zweimal mit 1 ml Ethanol gewaschen und sodann getrocknet.
**a.** N1-(1H-Imidazol-2-ylmethyl)-1,4-diamino-2-methyl-benzol Hydrochlorid und N4-(1H-Imidazol-2-ylmethyl)-1,4-diamino-2-methyl-benzol Hydrochlorid
   Verwendeter Aldehyd: Imidazol-2-carbaldehyd
   Ausbeute: 0,025g (40% der Theorie)
   Massenspketrum: MH+ 203(100)
b. N1-Furan-3-ylmethyl-1,4-diamino-2-methyl-benzol Hydrochlorid und
   N4-Furan-3-ylmethyl-1,4-diamino-2-methyl-benzol Hydrochlorid
   Verwendete Aldehydderivat: Furan-3-carbaldehyd
   Ausbeute: 0,025g (45% der Theorie)
   Massenspketrum: MH+ 203(100)
**c.** N1-Thiophen-2-ylmethyl-1,4-diamino-2-methyl-benzol Hydrochlorid und
   N4-Thiophen-2-ylmethyl-1,4-diamino-2-methyl-benzol Hydrochlorid
   Verwendete Aldehydderivat: Thiophen-2-carbaldehyd
   Ausbeute: 0,025g (42% der Theorie)
   Massenspketrum: MH+ 219(100)

**Beispiele 3:** Synthese von N1-Heteroarylmethyl-2-(2'-hydroxyethyl)-1,4-diaminobenzolen und N4-Heteroarylmethyl-2-(2'-hydroxyethyl)-1,4-diaminobenzolen 0,038 g (0,15 mmol) eine Mischung von N-(4-Amino-2-(2-hydroxyethyl)-phenyl)-carbaminsäure-tert-butylester und von N-(4-Amino-3-(2-hydroxyethyl)-phenyl)-carbaminsäure-tert-butylester und 0,10 mmol des entsprechenden Aldehyds werden in 1,2-Dichlorethan gelöst. Anschliessend werden 0,1 ml einer Essigsäurelösung (1-molar in 1,2-Dichlorethan) und 0,06 g NaBH(OAc)₃ (0,3 mmol) zugegeben und die Reaktionmischung wird 5 bis 15 Stunden bei Raumtemperatur gerührt. Nach Beendigung der Reaktion wird die Reaktionsmischung in 10 ml Essigsäureethylester gegossen, die organische Phase mit Natriumhydrogencarbonat extrahiert und sodann mit Magnesiumsulfat getrocknet. Das Lösungsmittel wird am Rotationsverdampfer abdestilliert und der Rückstand an Kieselgel mit Petrolether/Essigsäureethylester (9:1) gereinigt. Das so erhaltene Produkt wird in 4 ml Ethanol und 1,5 ml einer 2,9-molaren ethanolische Salzsäurelösung auf 50 °C erwärmt.
Der Niederschlag wird abfiltriert, zweimal mit 1 ml Ethanol gewaschen und sodann getrocknet.
**a.** 2-{5-Amino-2-[(thiophen-3-ylmethyl)-amino]-phenyl}-ethanol Hydrochlorid und 2-{6-Amino-3-[(thiophen-3-ylmethyl)-amino]-phenyl}ethanol Hydrochlorid
   Verwendete Aldehydderivat: Thiophen-3-carbaldehyd
   Ausbeute: 0,025g (38% der Theorie)
   Massenspketrum: MH+ 249(15), [M-Thienyl]+ 152(100)
**b.** 2-{5-Amino-2-[(thiophen-2-ylmethyl)-amino]-phenyl}-ethanol Hydrochlorid und 2-{6-Amino-3-[(thiophen-2-ylmethyl)-amino]-phenyl}ethanol Hydrochlorid
   Verwendete Aldehydderivat: Thiophen-2-carbaldehyd
   Ausbeute: 0,025g (38% der Theorie)
   Massenspketrum: MH+ 249(15) , [M-Thienyl]+ 152(100)

### Beispiele 4 bis 16: Haarfärbemittel

Es werden Haarfärbelösungen der folgenden Zusammensetzung hergestellt:

| | |
|---|---|
| 1,25 mmol | Entwicklersubstanz der Formel (I) gemäss Tabelle 1 |
| 1,25 mmol | Kupplersubstanz gemäß Tabelle 1 |
| 1,0 g | Kaliumoleat (8-prozentige wässrige Lösung) |
| 1,0 g | Ammoniak (22-prozentige wässrige Lösung) |
| 1,0 g | Ethanol |
| 0,3 g | Ascorbinsäure |
| ad 100,0 g | Wasser |

50 g der vorstehenden Färbelösung werden unmittelbar vor der Anwendung mit 50 g einer 6-prozentigen wässrigen Wasserstoffperoxidlösung vermischt. Anschliessend wird das Gemisch auf gebleichte Haare aufgetragen. Nach einer Einwirkungszeit von 30 Minuten bei 40 °C wird das Haar mit Wasser gespült, mit einem handelsüblichen Shampoo gewaschen und getrocknet. Die resultierenden Färbungen sind in Tabelle 1 zusammengefaßt.

**Tabelle 1:**

| **Beispiel Nr.** | **Entwickler- substanz der Formel (I)** | **Kupplersubstanz** | | | |
|---|---|---|---|---|---|
| | | **I.** **1,3-Di-hydroxy- benzol** | **II.** **1,3-Diamino-4- (2'-hydroxy- ethoxy)- benzol-sulfat** | **III.** **5-Amino-2- methyl-phenol** | **IV.** **1-Naphtol** |
| **4.** | gemäß Beispiel **1a** | braun | dunkelblau | purpur | blau |
| **5.** | gemäß Beispiel **1b** | braun | dunkelblau | purpur | blau |
| **6.** | gemäß Beispiel **1c** | dunkelblond | dunkelblau | purpur | blau |
| **7.** | gemäß Beispiel **1d** | dunkelblond | blau | purpur | violett |
| **8.** | gemäß Beispiel **1e** | dunkelblond | blau | purpur | grau |
| **9.** | gemäß Beispiel **1f** | dunkelblond | blau | purpur | blau |
| **10.** | gemäß Beispiel **1g** | dunkelblond | blau | purpur | blau |
| **11.** | gemäß Beispiel **1h** | hellbraun | grau | rot | braun |
| **12.** | gemäß Beispiel **2a** | mittelblond | blau | purpur | violett |
| **13.** | gemäß Beispiel **2b** | mittelblond | blau | purpur | violett |
| **14** | gemäß Beispiel **2c** | mittelblond | blau | purpur | violett |
| **15.** | gemäß Beispiel **3a** | hellblond | blau | purpur | blau |
| **16.** | gemäß Beispiel **3b** | hellblond | blau | purpur | blau |

### Beispiele 16 bis 56: Haarfärbemittel

Es werden Haarfärbelösungen der folgenden Zusammensetzung hergestellt:

| | |
|---|---|
| X g | N-Heteroarylmethyl-p-phenylendiamin der Formel (I) (Entwicklersubstanz **E1** bis **E4** gemäß Tabelle 2) |
| U g | Entwicklersubstanz **E8** bis **E15** gemäß Tabelle 2 |
| Y g | Kupplersubstanz **K11** bis **K36** gemäß Tabelle 4 |
| Z g | direktziehender Farbstoff **D1** bis **D3** gemäß Tabelle 3 |
| 10,000 g | Kaliumoleat (8-prozentige wässrige Lösung) |
| 10,000 g | Ammoniak (22-prozentige wässrige Lösung) |
| 10,000 g | Ethanol |
| 0,300 g | Ascorbinsäure |
| ad 100,000 g | Wasser |

30 g der vorstehenden Färbelösung werden unmittelbar vor der Anwendung mit 30 g einer 6-prozentigen wässsrigen Wasserstoffperoxidlösung vermischt. Anschliessend wird das Gemisch auf gebleichte Haare aufgetragen. Nach einer Einwirkungszeit von 30 Minuten bei 40 °Celsius wird das Haar mit Wasser gespült, mit einem handelsüblichen Shampoo gewaschen und getrocknet. Die Färbeergebnisse sind in Tabelle 5 zusammengefasst.

### Beispiele 57 bis 80: Haarfärbemittel

Es werden cremeförmige Farbträgermassen der folgenden Zusammensetzung hergestellt:

| | |
|---|---|
| X g | N-Heteroarylmethyl-p-phenylendiamin der Formel (I) (Entwicklersubstanz **E1** bis **E4** gemäß Tabelle 2) |
| U g | Entwicklersubstanz **E8** bis **E15** gemäß Tabelle 2 |
| Y g | Kupplersubstanz **K11** bis **K36** gemäss Tabelle 4 |
| Z g | direktziehender Farbstoff **D2** gemäss Tabelle 3 |
| 15,0 g | Cetylalkohol |
| 0,3 g | Ascorbinsäure |
| 3,5 g | Natriumlaurylalkoholdiglycolethersulfat, 28-prozentige wässrige Lösung |
| 3,0 g | Ammoniak, 22-prozentige wässrige Lösung |
| 0,3 g | Natriumsulfit, wasserfrei |
| ad 100,0 g | Wasser |

30 g der vorstehenden Färbecreme werden unmittelbar vor der Anwendung mit 30 g einer 6-prozentigen Wasserstoffperoxidlösung vermischt. Anschliessend wird das Gemisch auf das Haar aufgetragen. Nach einer Einwirkzeit von 30 Minuten wird das Haar mit Wasser gespült, mit einem handelsüblichen Shampoo gewaschen und getrocknet. Die Färbeergebnisse sind in Tabelle 6 zusammengefasst.

**Tabelle 2:**

| **Entwicklersubstanzen** | |
|---|---|
| **E1** | N-Thiophen-3-ylmethyl-1,4-diamino-benzol Hydrochlorid |
| **E2** | N-Furan-3-ylmethyl-1,4-diamino-benzol Hydrochlorid |
| **E3** | N-(1H-Imidazol-2-ylmethyl)-1,4-diamino-benzol Hydrochlorid |
| **E4** | N-Thiophen-2-ylmethyl-1,4-diamino-benzol Hydrochlorid |
| | |
| **E8** | 1,4-Diaminobenzol |
| **E9** | 2,5-Diamino-phenylethanol-sulfat |
| **E10** | 3-Methyl-4-amino-phenol |
| **E11** | 4-Amino-2-aminomethyl-phenol-dihydrochlorid |
| **E12** | 4-Amino-phenol |
| **E13** | N,N-Bis(2'-hydroxyethyl)-p-phenylendiamin-sulfat |
| **E14** | 4,5-Diamino-1-(2'-hydroxyethyl)-pyrazol-sulfat |
| **E15** | 2,5-Diaminotoluol-sulfat |

**Tabelle 3:**

| **Direktziehende Farbstoffe** | |
|---|---|
| **D1** | 2,6-Diamino-3-((pyridin-3-yl)azo)pyridin |
| **D2** | 6-Chlor-2-ethylamino-4-nitro-phenol |
| **D3** | 2-Amino-6-chlor-4-nitro-phenol |

**Tabelle 4:**

| **Kupplersubstanzen** | |
|---|---|
| **K11** | 1,3-Diaminobenzol |
| **K12** | 2-Amino-4-(2'-hydroxyethyl)amino-anisol-sulfat |
| **K13** | 1,3-Diamino-4-(2'-hydroxyethoxy)benzol-sulfat |
| **K14** | 2,4-Diamino-5-fluor-toluol-sulfat |
| **K15** | 3-Amino-2-methylamino-6-methoxy-pyridin |
| **K16** | 3,5-Diamino-2,6-dimethoxy-pyridin-dihydrochlorid |
| **K17** | 2,4-Diamino-5-ethoxy-toluol-sulfat |
| **K18** | N-(3-Dimethylamino)phenylharnstoff |
| **K19** | 1,3-Bis(2,4-Diaminophenoxy)propan-tetrahydrochlorid |
| **K21** | 3-Amino-phenol |
| **K22** | 5-Amino-2-methyl-phenol |
| **K23** | 3-Amino-2-chlor-6-methyl-phenol |
| **K24** | 5-Amino-4-fluor-2-methyl-phenol-sulfat |
| **K25** | 1-Naphthol |
| **K26** | 1-Acetoxy-2-methyl-naphthalin |
| **K31** | 1,3-Dihydroxy-benzol |
| **K32** | 2-Methyl-1,3-dihydroxy-benzol |
| **K33** | 1-Chlor-2,4-dihydroxy-benzol |
| **K34** | 4-(2'-Hydroxyethyl)amino-1,2-methylendioxybenzol-hydrochlorid |
| **K35** | 3,4-Methylendioxy-phenol |
| **K36** | 2-Amino-5-methyl-phenol |

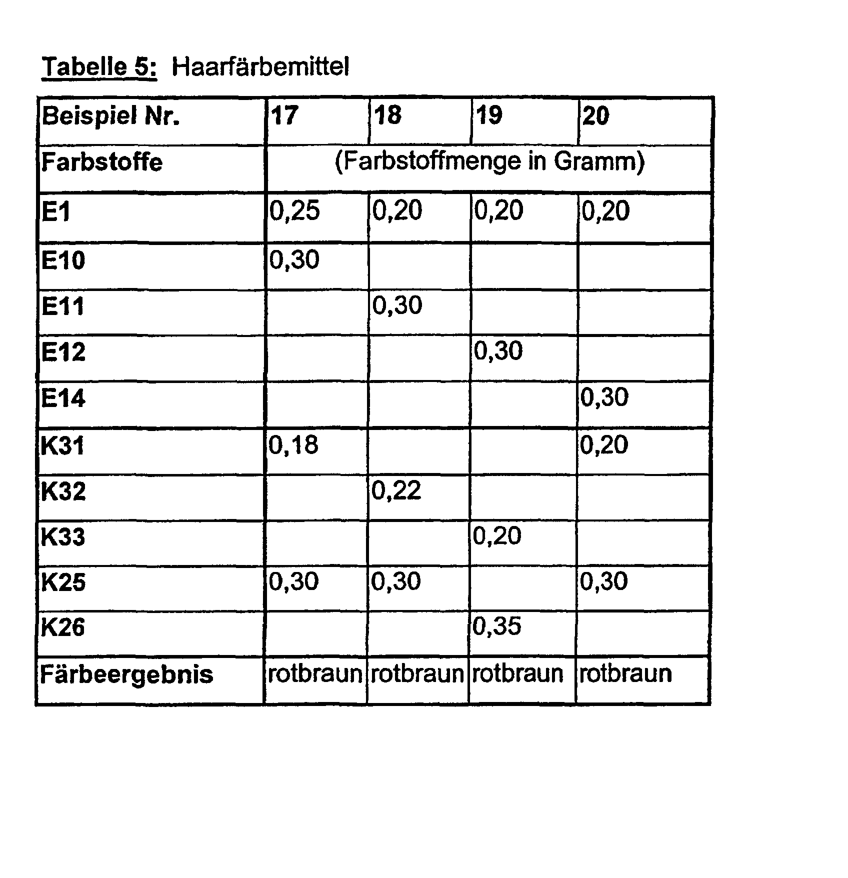

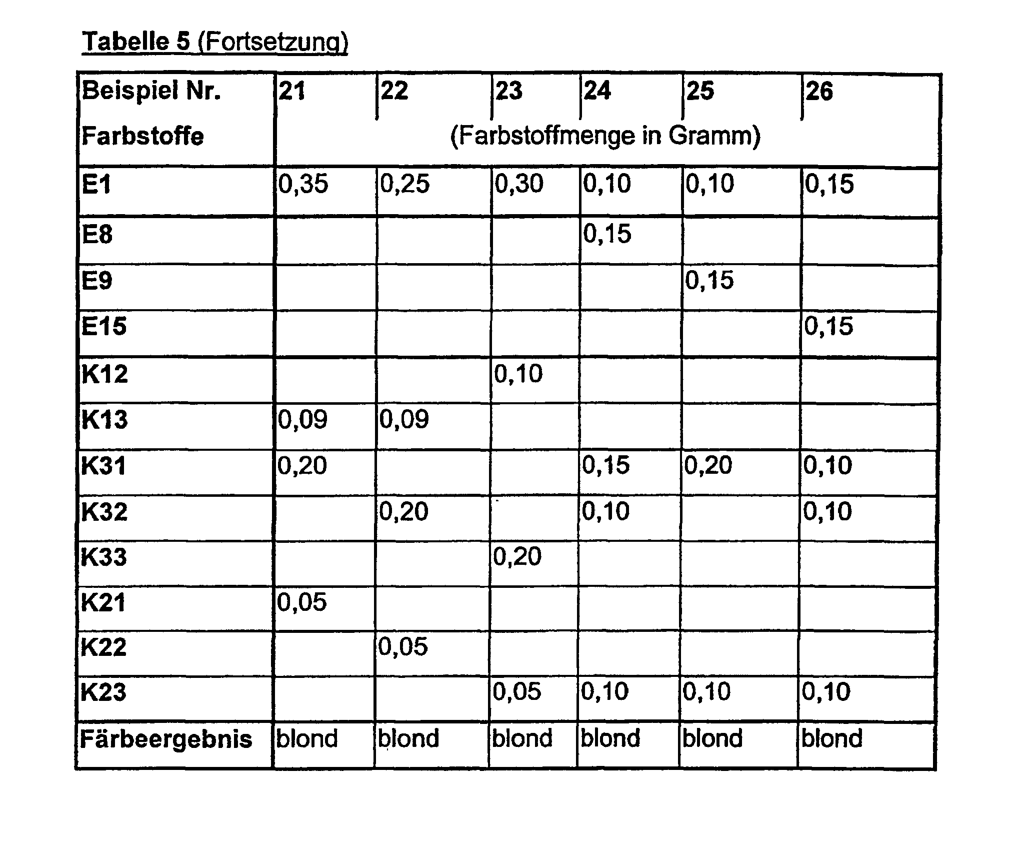

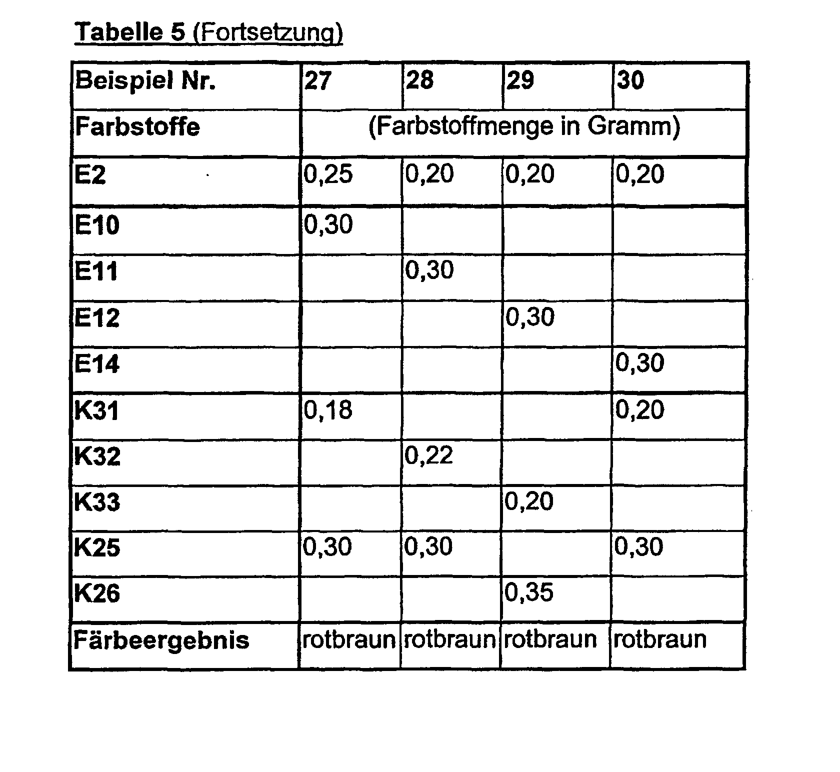

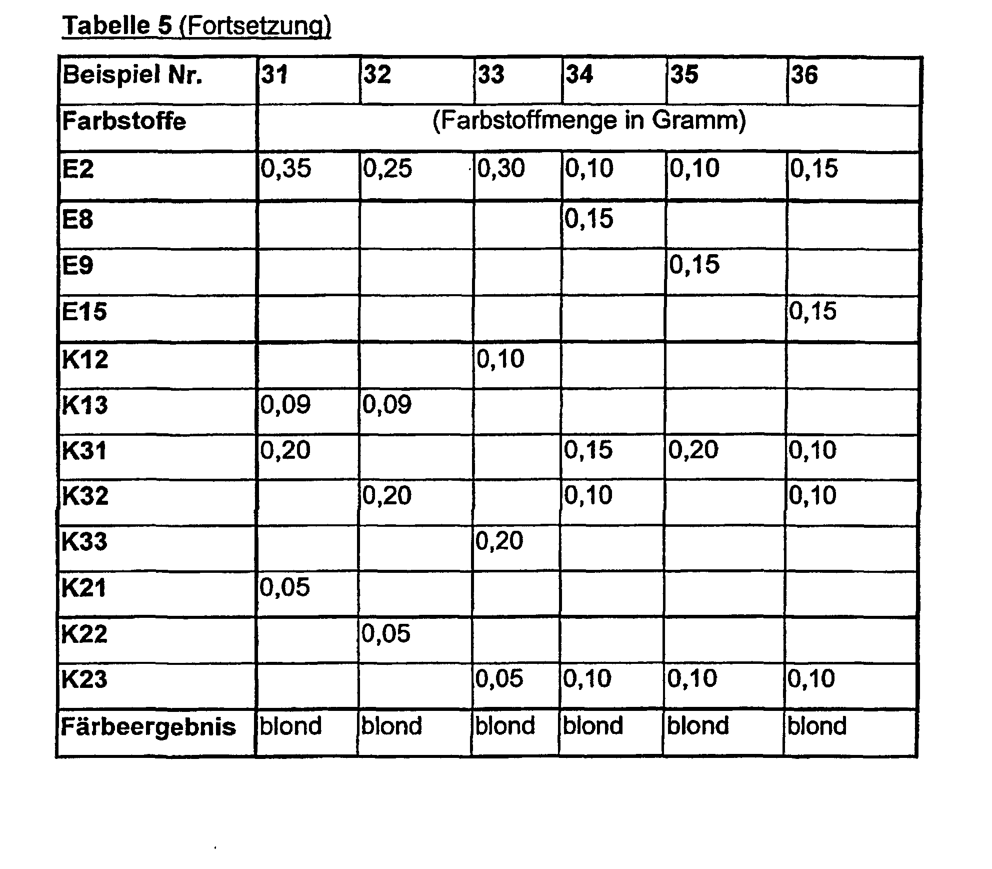

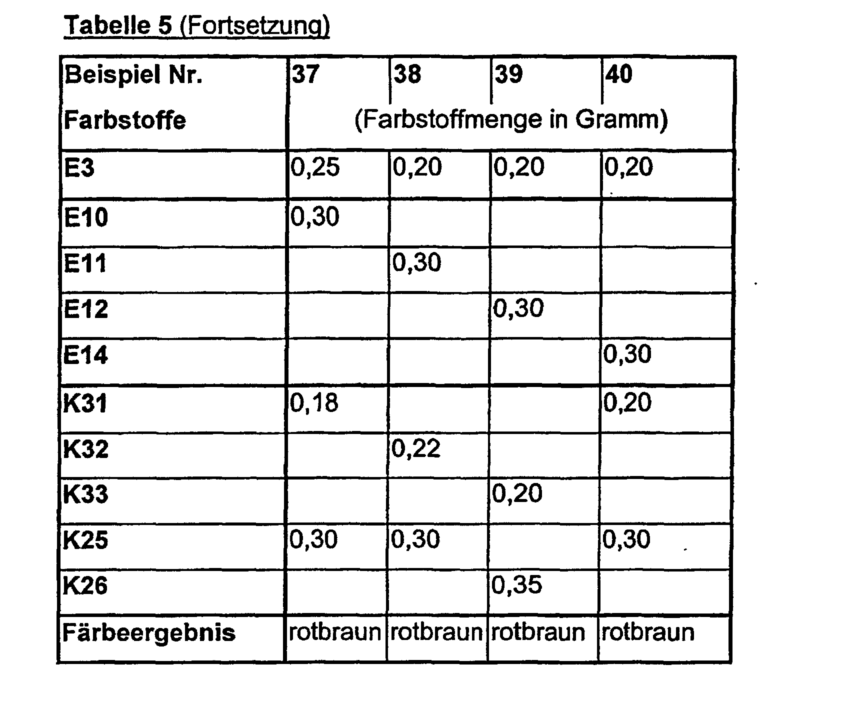

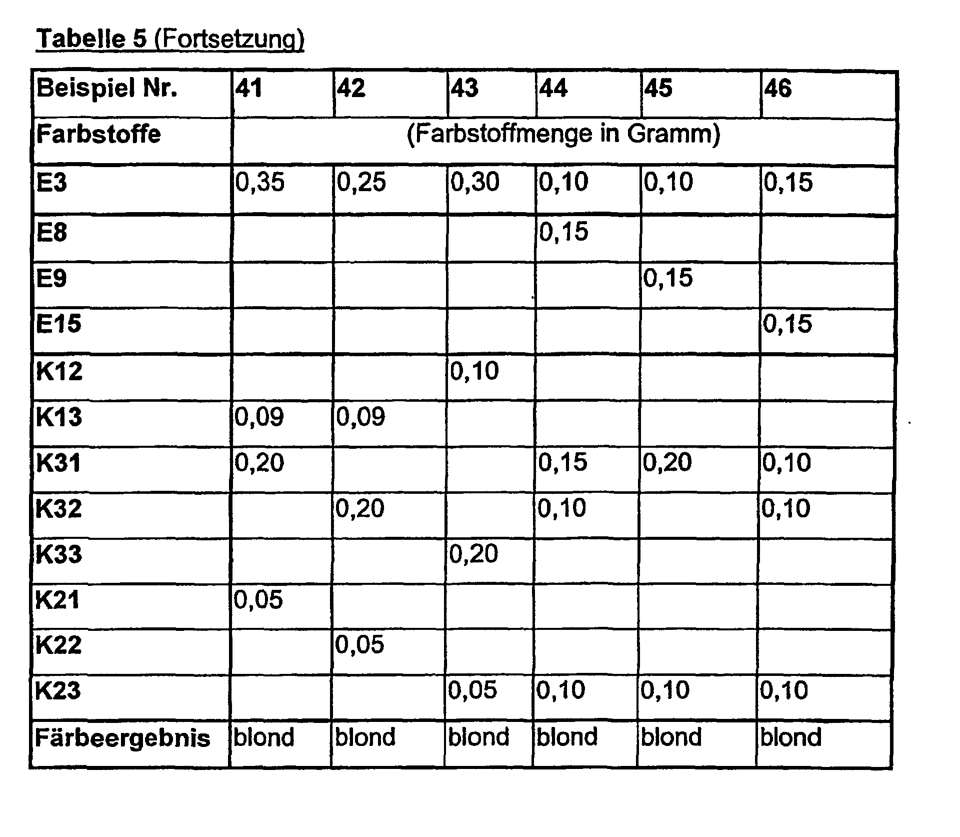

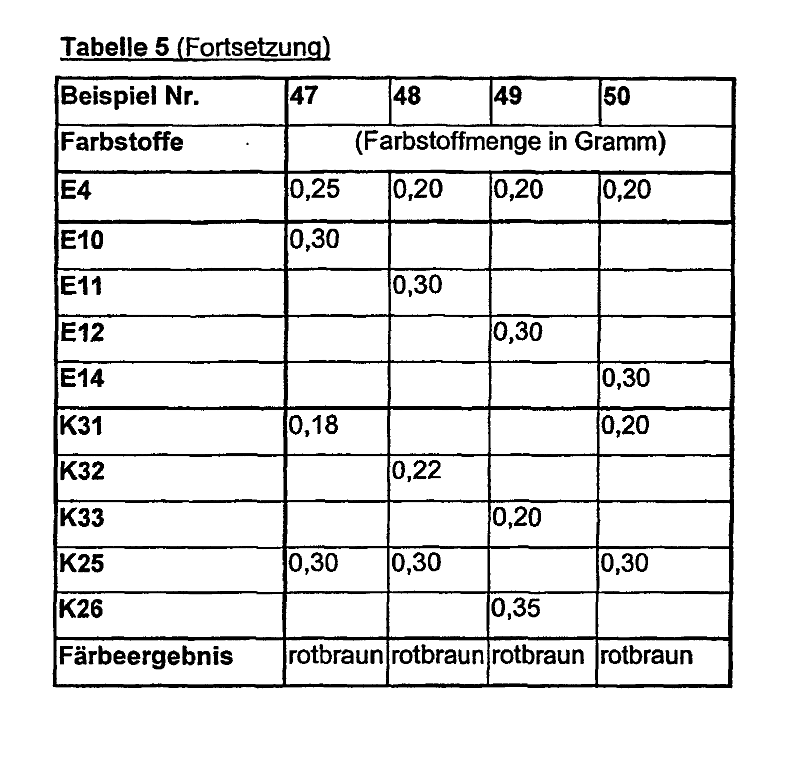

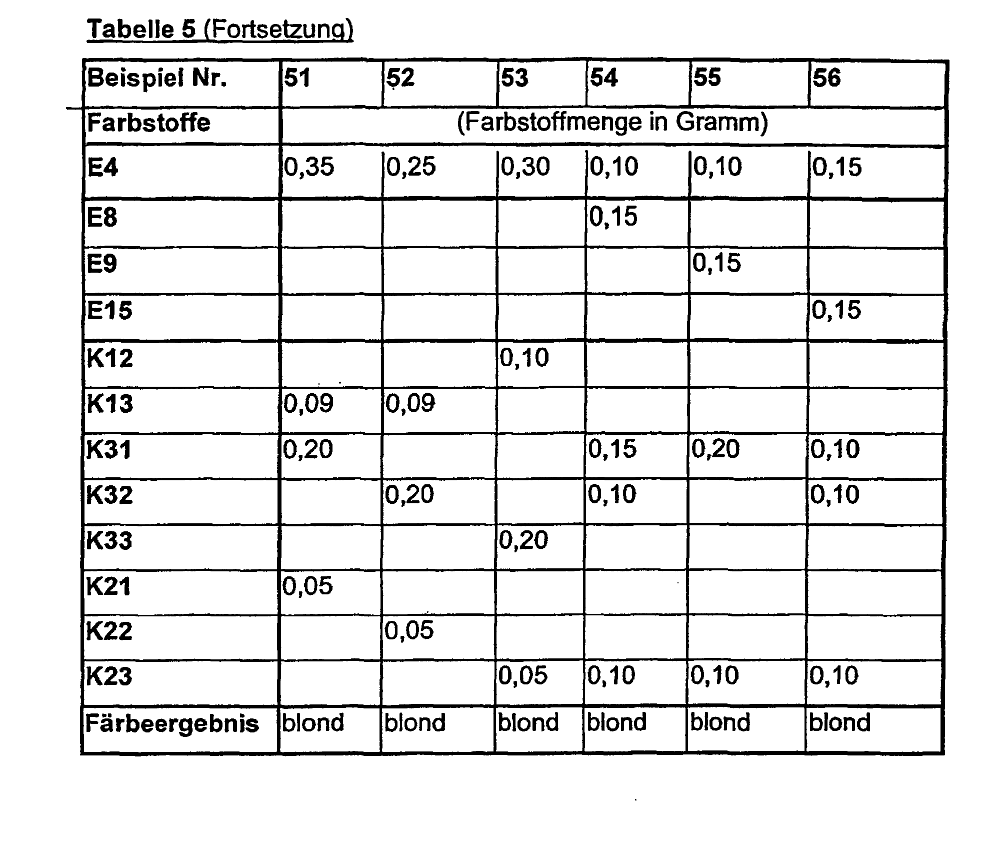

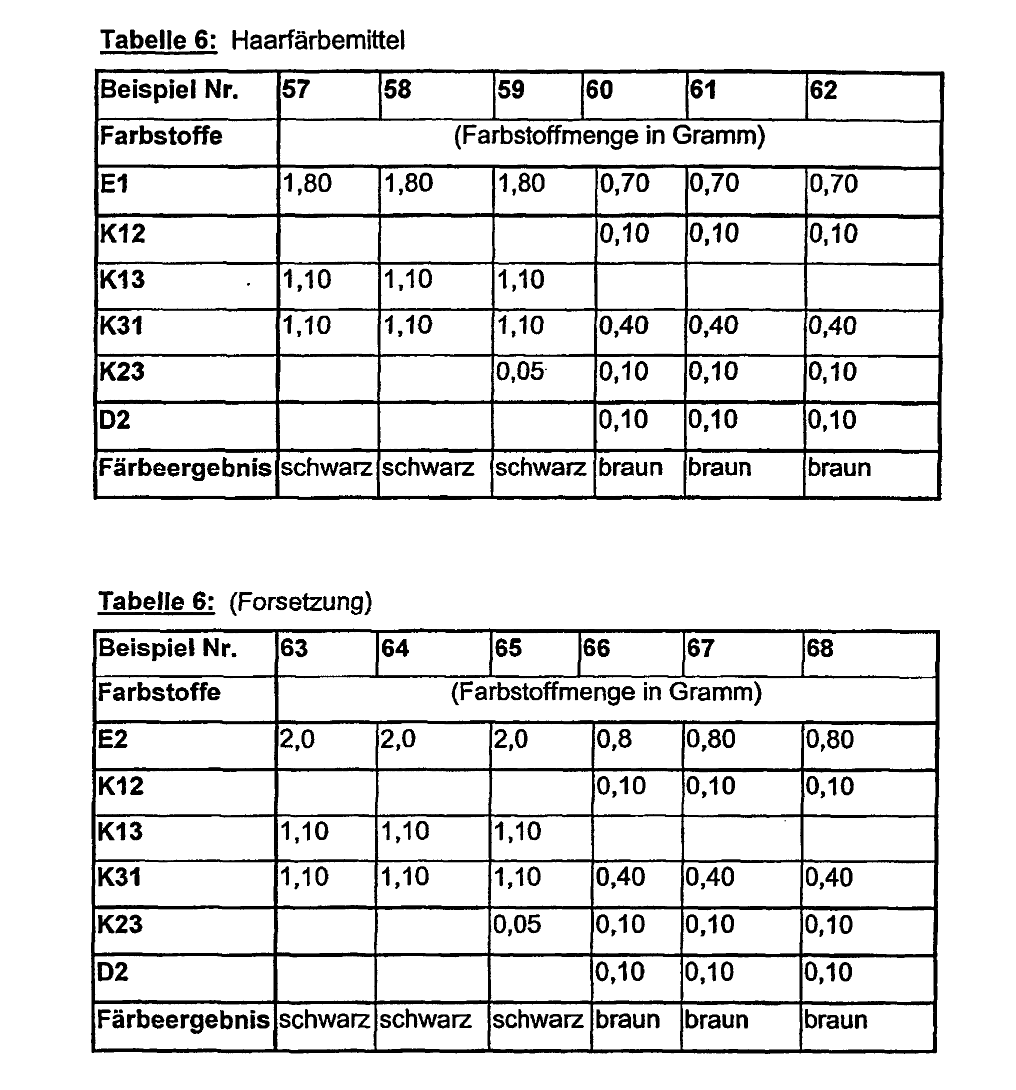

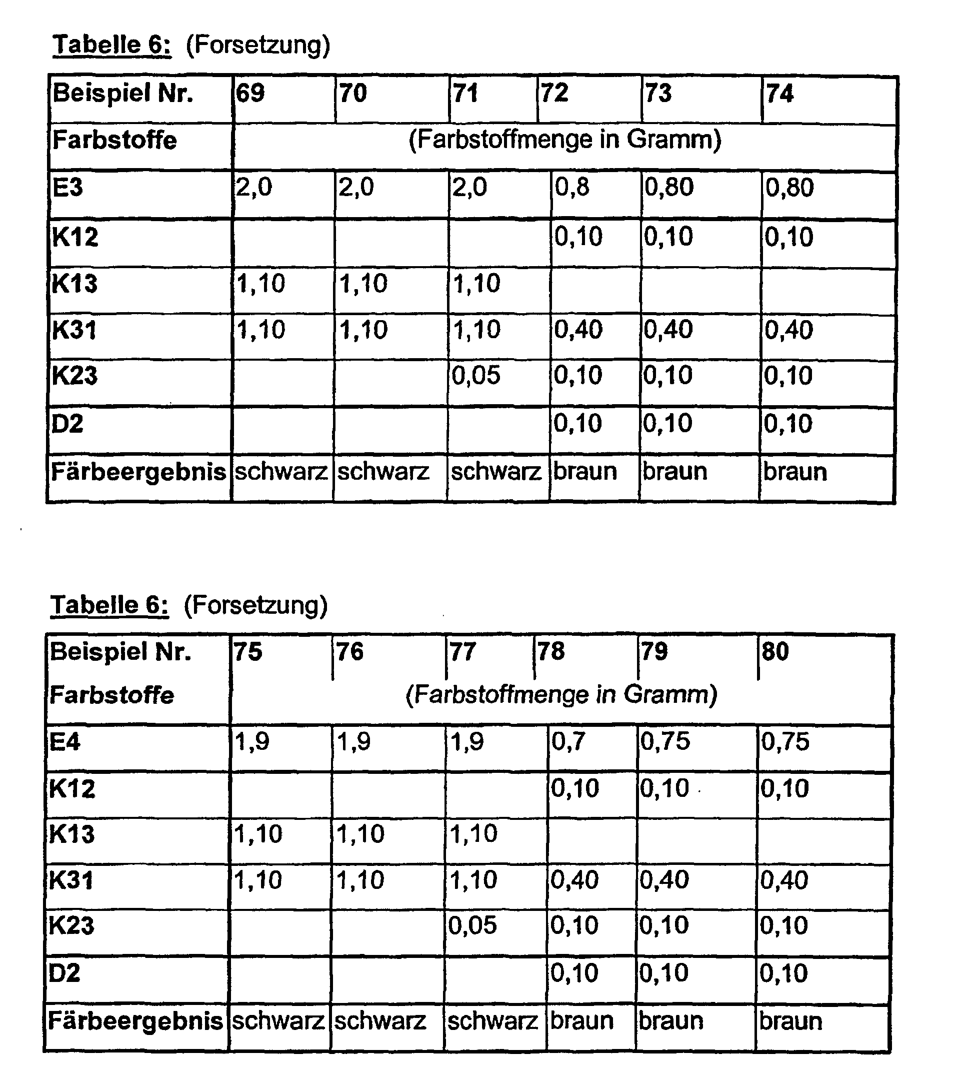

Alle Prozentangaben stellen, soweit nicht anders angegeben, Gewichtsprozente dar.

## Patentansprüche

1. N-Heteroarylmethyl-p-phenylendiamin-Derivate der allgemeinen Formel (I) oder deren physiologisch verträgliche, wasserlösliche Salze, 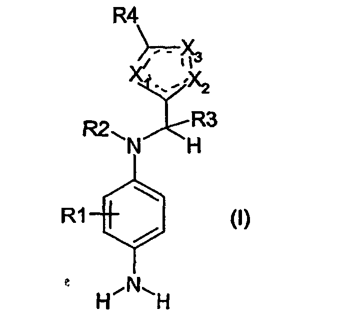 worin
**X1** gleich Schwefel, Stickstoff, C-R6 oder N-R5 ist;
**X2** gleich Schwefel, Stickstoff, C-R6 oder N-R5 ist
**X3** gleich Schwefel, Stickstoff, Sauerstoff, C-R6 oder N-R5 ist;
**R1** gleich Wasserstoff, einer C₁-C₄-Alkylgruppe oder einer C₁-C₄-Hydroxyalkylgruppe ist;
**R2,R3** gleich oder verschieden sein können und unabhängig voneinander gleich Wasserstoff oder einer C₁-C₆-Alkylgruppe sind;
R4,R6 gleich oder verschieden sein können und unabhängig voneinander gleich Wasserstoff, einem Halogenatom, einer Cyanogruppe, einer C₁-C₄-Alkoxygruppe, einer C₁-C₆-Alkylgruppe, einer C₁-C₄-Alkylthioethergruppe, einer Mercaptogruppe, einer Nitrogruppe, einer Aminogruppe, einer C₁-C₄-Alkylaminogruppe, einer Di(C₁-C₄)-alkylaminogruppe, einer Di(C₁-C₄-hydroxy-alkyl)aminogruppe, einer C₁-C₄-Hydroxyalkylamino-gruppe, einer Trifluormethangruppe, einer -C(O)CH₃-Gruppe, einer -C(O)CF₃-Gruppe, einer -Si(CH₃)₃-Gruppe, einer C₁-C₄-Hydroxyalkylgruppe oder einer C₃-C₄ Dihydroxyalkylgruppe sind;
**R5** gleich Wasserstoff, einer C₁-C₆-Alkylgruppe, einer C₁-C₄-Hydroxyalkylgruppe, einer Phenylgruppe oder einer Acetylgruppe ist;
wobei mindestens einer und maximal zwei der Reste **X1** bis **X3** gleich C-R6 sind und maximal einer der Reste **X1** bis **X3** gleich Schwefel, Sauerstoff oder N-R5 ist.

2. N-Heteroarylmethyl-p-phenylendiamin-Derivat gemäß Anspruch 1, **dadurch gekennzeichnet, dass** (i) **R1, R2** und **R3** oder **R1, R2, R3** und **R4** Wasserstoff bedeuten oder (ii) **R1, R2** und **R3** oder **R1, R2, R3** und **R4** Wasserstoff bedeuten und **X1** gleich Schwefel oder N-H ist und **X2** gleich Stickstoff oder C-R6 ist und **X3** gleich C-R6 ist oder (iii) **R1, R2** und **R3** oder **R1, R2, R3** und **R4** gleich Wasserstoff sind und **X3** gleich Schwefel oder Sauerstoff ist und **X1** und **X2** gleich C-R6 sind oder (iv) **R1, R2** und **R3** oder **R1, R2, R3** und **R4** gleich Wasserstoff sind und **X2** gleich Schwefel oder Sauerstoff ist und **X1** und **X3** gleich C-R6 sind.

3. N-Heteroarylmethyl-p-phenylendiamin-Derivat gemäß Anspruch 1 oder 2, **dadurch gekennzeichnet, dass** das N-Heteroarylmethyl-p-phenylendiamin-Derivat der Formel (I) ausgewählt ist aus N-Thiophen-3-ylmethyl-1,4-diamino-benzol, N-Furan-3-ylmethyl-1,4-diamino-benzol, N-(1 H-Imidazol-2-ylmethyl)-1,4-diamino-benzol, N-(1H-Pyrrol-2-ylmethyl)-1,4-diamino-benzol und N-Thiophen-2-ylmethyl-1,4-diamino-benzol oder deren physiologisch verträglichen Salzen.

4. Mittel zum oxidativen Färben von Keratinfasem auf der Basis einer Entwicklersubstanz-Kupplersubstanz-Kombination, **dadurch gekennzeichnet, dass** es als Entwicklersubstanz mindestens ein N-Heteroarylmethyl-p-phenylendiamin-Derivat der Formel (I) nach einem der Ansprüche 1 bis 3 enthält.

5. Mittel nach Anspruch 4, **dadurch gekennzeichnet, dass** es die Verbindung der Formel (I) in einer Menge von 0,005 bis 20 Gewichtsprozent enthält.

6. Mittel nach Anspruch 4 oder 5, **dadurch gekennzeichnet, dass** die Kupplersubstanz ausgewählt ist aus der Gruppe bestehend aus 2,6-Diamino-pyridin, 2-Amino-4-[(2-hydroxyethyl)amino]-anisol, 2,4-Diamino-1-fluor-5-methyl-benzol, 2,4-Diamino-1-methoxy-5-methylbenzol, 2,4-Diamino-1-ethoxy-5-methyl-benzol, 2,4-Diamino-1-(2-hydroxyethoxy)-5-methyl-benzol, 2,4-Di[(2-hydroxyethyl)amino]-1,5-dimethoxy-benzol, 2,3-Diamino-6-methoxy-pyridin, 3-Amino-6-methoxy-2-(methylamino)-pyridin, 2,6-Diamino-3,5-dimethoxy-pyridin, 3,5-Diamino-2,6-dimethoxy-pyridin, 1,3-Diamino-benzol, 2,4-Diamino-1-(2-hydroxyethoxy)-benzol, 2,4-Diamino-1,5-di(2-hydroxyethoxy)-benzol, 1-(2-Aminoethoxy)-2,4-diamino-benzol, 2-Amino-1-(2-hydroxyethoxy)-4-methylaminobenzol, 2,4-Diaminophenoxy-essigsäure, 3-[Di(2-hydroxyethyl)amino]-anilin, 4-Amino-2-di[(2-hydroxyethyl)amino]-1-ethoxy-benzol, 5-Methyl-2-(1-methylethyl)-phenol, 3-[(2-Hydroxyethyl)amino]-anilin, 3-[(2-Aminoethyl)amino]-anilin, 1,3-Di(2,4-diaminophenoxy)-propan, Di(2,4-diaminophenoxy)-methan, 1,3-Diamino-2,4-dimethoxy-benzol, 2,6-Bis(2-hydroxyethyl)amino-toluol, 4-Hydroxyindol, 3-Dimethylamino-phenol, 3-Diethylamino-phenol, 5-Amino-2-methyl-phenol, 5-Amino-4-fluor-2-methylphenol, 5-Amino-4-methoxy-2-methyl-phenol, 5-Amino-4-ethoxy-2-methylphenol, 3-Amino-2,4-dichlor-phenol, 5-Amino-2,4-dichlor-phenol, 3-Amino-2-methyl-phenol, 3-Amino-2-chlor-6-methyl-phenol, 3-Amino-phenol, 2-[(3-Hydroxyphenyl)amino]-acetamid, 5-[(2-Hydroxyethyl)amino]-2-methyl-phenol, 3-[(2-Hydroxyethyl)amino]-phenol, 3-[(2-Methoxyethyl)-amino]-phenol, 5-Amino-2-ethyl-phenol, 2-(4-Amino-2-hydroxyphenoxy)-ethanol, 5-[(3-Hydroxypropyl)amino]-2-methyl-phenol, 3-[(2,3-Dihydroxypropyl)amino]-2-methyl-phenol, 3-[(2-Hydroxyethyl)amino]-2-methylphenol, 2-Amino-3-hydroxy-pyridin, 5-Amino-4-chlor-2-methyl-phenol, 1-Naphthol, 1,5-Dihydroxy-naphthalin, 1,7-Dihydroxy-naphthalin, 2,3-Dihydroxy-naphthalin, 2,7-Dihydroxy-naphthalin, 2-Methyl-1-naphtholacetat, 1,3-Dihydroxy-benzol, 1-Chlor-2,4-dihydroxy-benzol, 2-Chlor-1,3-dihydroxy-benzol, 1,2-Dichlor-3,5-dihydroxy-4-methyl-benzol, 1,5-Dichlor-2,4-dihydroxy-benzol, 1,3-Dihydroxy-2-methyl-benzol, 3,4-Methylendioxy-phenol, 3,4-Methylendioxy-anilin, 5-[(2-Hydroxyethyl)amino]-1,3-benzodioxol, 6-Brom-1-hydroxy-3,4-methylendioxy-benzol, 3,4-Diaminobenzoesäure, 3,4-Dihydro-6-hydroxy-1,4(2H)-benzoxazin, 6-Amino-3,4-dihydro-1,4(2H)-benzoxazin, 3-Methyl-1-phenyl-5-pyrazolon, 5,6-Dihydroxy-indol, 5,6-Dihydroxy-indolin, 5-Hydroxy-indol, 6-Hydroxy-indol, 7-Hydroxy-indol und 2,3-Indolindion.

7. Mittel nach einem der Ansprüche 4 bis 6, **dadurch gekennzeichnet, dass** die Entwicklersubstanzen und Kupplersubstanzen, bezogen auf die Gesamtmenge des Färbemittels, jeweils in einer Gesamtmenge von 0,005 bis 20 Gewichtsprozent enthalten sind.

8. Mittel nach einem der Ansprüche 4 bis 7 , **dadurch gekennzeichnet, dass** es zusätzlich mindestens einen direktziehenden Farbstoff enthält.

9. Mittel nach einem der Ansprüche 4 bis 8, **dadurch gekennzeichnet, dass** es ein Haarfärbemittel ist.

## Claims

1. N-Heteroarylmethyl-p-phenylenediamine derivatives of the general formula (I) or physiologically compatible, water-soluble salts thereof, 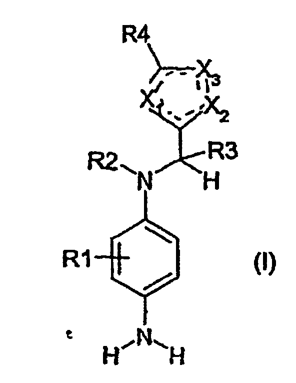 in which
**X1** is sulphur, nitrogen, C-R6 or N-R5;
**X2** is sulphur, nitrogen, C-R6 or N-R5;
**X3** is sulphur, nitrogen, oxygen, C-R6 or N-R5;
**R1** is hydrogen, a C₁-C₄-alkyl group or a C₁-C₄-hydroxyalkyl group;
**R2, R3** may be identical or different and, independently of one another, are hydrogen or a C₁-C₆-alkyl group;
**R5, R6** may be identical or different and, independently of one another, are hydrogen, a halogen atom, a cyano group, a C₁-C₄-alkoxy group, a C₁-C₆-alkyl group, a C₁-C₄-alkylthio ether group, a mercapto group, a nitro group, an amino group, a C₁-C₄-alkylamino group, a di(C₁-C₄)alkyl-amino group, a di(C₁-C₄-hydroxyalkyl)-amino group, a C₁-C₄-hydroxyalkylamino group, a trifluoromethane group, a -C(O)CH₃ group, a -C(O)CF₃ group, a -Si(CH₃)₃ group, a C₁-C₄-hydroxyalkyl group or a C₃-C₄-dihydroxyalkyl group;
**R5** is hydrogen, a C₁-C₆-alkyl group, a C₁-C₄-hydroxyalkyl group, a phenyl group or an acetyl group;
where at least one and at most two of the radicals **X1** to **X3** are C-R6, and at most one of the radicals **X1** to **X3** is sulphur, oxygen or N-R5.

2. N-Heteroarylmethyl-p-phenylenediamine derivative according to Claim 1, **characterized in that** (i) **R1**, **R2** and **R3** or **R1**, **R2**, **R3** and **R4** are hydrogen or (ii) **R1**, **R2** and **R3** or **R1**, **R2**, **R3** and **R4** are hydrogen and **X1** is sulphur or is N-H and **X2** is nitrogen or C-R6 and **X3** is C-R6 or (iii) **R1**, **R2** and **R3** or **R1**, **R2**, **R3** and **R4** are hydrogen and **X3** is sulphur or oxygen and **X1** and **X2** are C-R6 or (iv) **R1**, **R2** and **R3** or **R1**, **R2**, **R3** and **R4** are hydrogen and **X2** is sulphur or oxygen and **X1** and **X3** are C-R6.

3. N-Heteroarylmethyl-p-phenylenediamine derivative according to Claim 1 or 2, **characterized in that** the N-heteroarylmethyl-p-phenylenediamine derivative of the formula (I) is chosen from N-thiophen-3-ylmethyl-1,4-diaminobenzene, N-furan-3-ylmethyl-1,4-diaminobenzene, N-(1H-imidazol-2-ylmethyl)-1,4-diaminobenzene, N-(1H-pyrrol-2-ylmethyl)-1,4-diaminobenzene and N-thiophen-2-ylmethyl-1,4-diaminobenzene or physiologically compatible salts thereof.

4. Agent for the oxidative colouring of keratin fibres based on a developer substance/coupler substance combination, **characterized in that** it comprises, as developer substance, at least one N-heteroarylmethyl-p-phenylenediamine derivative of the formula (I) as claimed in one of Claims 1 to 3.

5. Agent according to Claim 4, **characterized in that** it comprises the compound of the formula (I) in an amount of from 0.005 to 20 percent by weight.

6. Agent according to Claim 4 or 5, **characterized in that** the coupler substance is chosen from the group consisting of 2,6-diaminopyridine, 2-amino-4-[(2-hydroxyethyl)amino]anisole, 2,4-diamino-1-fluoro-5-methylbenzene, 2,4-diamino-1-methoxy-5-methylbenzene, 2,4-diamino-1-ethoxy-5-methylbenzene, 2,4-diamino-1-(2-hydroxyethoxy)-5-methylbenzene, 2,4-di[(2-hydroxyethyl)amino]-1,5-dimethoxybenzene, 2,3-diamino-6-methoxypyridine, 3-amino-6-methoxy-2-(methylamino)pyridine, 2,6-diamino-3,5-dimethoxypyridine, 3,5-diamino-2,6-dimethoxypyridine, 1,3-diaminobenzene, 2,4-diamino-1-(2-hydroxyethoxy)benzene, 2,4-diamino-1,5-di-(2-hydroxyethoxy)benzene, 1,-(2-aminoethoxy)-2,4-diaminobenzene, 2-amino-1-(2-hydroxyethoxy)-4-methylaminobenzene, 2,4-diaminophenoxyacetic acid, 3-[di(2-hydroxyethyl)amino]aniline, 4-amino-2-di[(2-hydroxyethyl)amino]-1-ethoxybenzene, 5-methyl-2-(1-methylethyl)phenol, 3-[(2-hydroxy-ethyl)amino]aniline, 3-[(2-aminoethyl)amino]-aniline, 1,3-di(2,4-diaminophenoxy)propane, di(2,4-diaminophenoxy)methane, 1,3-diamino-2,4-di-methoxybenzene, 2,6-bis(2-hydroxyethyl)amino-toluene, 4-hydroxyindole, 3-dimethylaminophenol, 3-diethylaminophenol, 5-amino-2-methylphenol, 5-amino-4-fluoro-2-methylphenol, 5-amino-4-methoxy-2-methylphenol, 5-amino-4-ethoxy-2-methylphenol, 3-amino-2,4-dichlorophenol, 5-amino-2,4-dichlorophenol, 3-amino-2-methyl-phenol, 3-amino-2-chloro-6-methylphenol, 3-amino-phenol, 2-[(3-hydroxyphenyl)amino]acetamide, 5-[(2-hydroxyethyl)amino]-2-methylphenol, 3-[(2-hydroxyethyl)amino]phenol, 3-[(2-methoxy-ethyl)amino]phenol, 5-amino-2-ethylphenol, 2-(4-amino-2-hydroxyphenoxy)ethanol, 5-[(3-hydroxypropyl)amino]-2-methylphenol, 3-[(2,3-dihydroxypropyl)amino]-2-methylphenol, 3-[(2-hydroxyethyl)amino]-2-methylphenol, 2-amino-3-hydroxypyridine, 5-amino-4-chloro-2-methyl-phenol, 1-naphthol, 1,5-dihydroxynaphthalene, 1,7-dihydroxynaphthalene, 2,3-dihydroxynaphthalene, 2,7-dihydroxynaphthalene, 2-methyl-1-naphthol acetate, 1,3-dihydroxybenzene, 1-chloro-2,4-dihydroxybenzene, 2-chloro-1,3-di-hydroxybenzene, 1,2-dichloro-3,5-dihydroxy-4-methylbenzene, 1,5-dichloro-2,4-dihydroxy-benzene, 1,3-dihydroxy-2-methylbenzene, 3,4-methylenedioxyphenol, 3,4-methylenedioxyaniline, 5-[(2-hydroxyethyl)amino]-1,3-benzo-dioxole, 6-bromo-1-hydroxy-3,4-methylenedioxybenzene, 3,4-diaminobenzoic acid, 3,4-dihydro-6-hydroxy-1,4(2H)benzoxazine, 6-amino-3,4-dihydro-1,4(2H)-benzoxazine, 3-methyl-1-phenyl-5-pyrazolone, 5,6-dihydroxyindole, 5,6-dihydroxyindoline, 5-hydroxyindole, 6-hydroxyindole, 7-hydroxyindole and 2,3-indolinedione.

7. Agent according to one of Claims 4 to 6, **characterized in that** the developer substances and coupler substances are in each case present in a total amount of from 0.005 to 20 percent by weight, based on the total amount of the colorant.

8. Agent according to one of Claims 4 to 7, **characterized in that** it additionally comprises at least one direct dye.

9. Agent according to one of Claims 4 to 8, **characterized in that** it is a hair colorant.

## Revendications

1. Dérivés de N-hétéroarylméthyl-p-phénylènediamine de formule générale (I) ou sels hydrosolubles physiologiquement acceptables, de tels dérivés 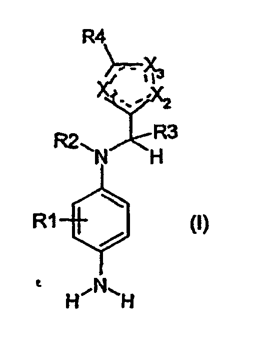 formule dans laquelle
**X1** représente un atome de soufre ou d'azote, C-R6 ou N-R5 ;
**X2** représente un atome de soufre ou d'azote, C-R6 ou N-R5 ;
X3 représente un atome de soufre, d'azote ou d'oxygène ou C-R6 ou N-R5 ;
**R1** représente un atome d'hydrogène, un groupe alkyle en C₁-C₄ ou un groupe hydroxyalkyle en C₁-C₄ ;
**R2**, **R3** peuvent être identiques ou différents et représentent, indépendamment l'un de l'autre, un atome d'hydrogène ou un groupe alkyle en C₁-C₆ ;
**R4, R6** peuvent être identiques ou différents et représentent, indépendamment l'un de l'autre, un atome d'hydrogène, un atome d'halogène, un groupe cyano, un groupe alcoxy en C₁-C₄, un groupe alkyle en C₁-C₆, un groupe alkyl-(C₁-C₄)thioéther, un groupe mercapto, un groupe nitro, un groupe amino, un groupe alkyl(C₁-C₄)amino, un groupe dialkyl(C₁-C₄)-amino, un groupe di[hydroxyalkyl(C₁-C₄)amino], un groupe hydroxyalkyl(C₁-C₄)amino, un groupe trifluorométhane, un groupe -C(O)CH₃, un groupe -C(O)CF₃, un groupe -Si(CH₃)₃, un groupe hydroxyalkyle en C₁-C₄ ou un groupe dihydroxyalkyle en C₃-C₄ ;
**R5** représente un atome d'hydrogène, un groupe alkyle en C₁-C₆, un groupe hydroxyalkyle en C₁-C₄, un groupe phényle ou un groupe acétyle ;
au moins un et au maximum deux des radicaux **X1** à **X3** représentant C-R6 et au maximum un des radicaux **X1** à **X3** étant un atome de soufre ou d'oxygène ou N-R5.

2. Dérivé de N-hétéroarylméthyl-p-phénylènediamine selon la revendication 1, **caractérisé en ce que** (i) **R1**, **R2** et **R3** ou **R1**, **R2**, **R3** et **R4** représentent des atomes d'hydrogène ou (ii) **R1**, **R2** et **R3** ou **R1**, **R2**, **R3** et **R4** représentent des atomes d'hydrogène et **X1** est un atome de soufre ou N-H et **X2** est un atome d'azote ou C-R6, et **X3** est C-R6 ou (iii) **R1**, **R2** et **R3** ou **R1**, **R2**, **R3** et **R4** sont des atomes d'hydrogène et **X3** est un atome de soufre ou d'oxygène et **X1** et **X2** représentent C-R6 ou (iv) **R1**, **R2** et **R3** ou **R1**, **R2**, **R3** et **R4** sont des atomes d'hydrogène et **X2** est un atome de soufre ou d'oxygène et **X1** et **X3** représentent C-R6.

3. Dérivé de N-hétéroarylméthyl-p-phénylènediamine selon la revendication 1 ou 2, **caractérisé en ce que** le dérivé de N-hétéroarylméthyl-p-phénylènediamine de formule (I) est choisi parmi le N-thiophén-3-ylméthyl-1,4-diaminobenzène, le N-furann-3-ylméthyl-1,4-diaminobenzène, le N-(1H-imidazol-2-ylméthyl)-1,4-diaminobenzène, le N-(1H-pyrrol-2-ylméthyl)-1,4-diaminobenzène et le N-thiophén-2-ylméthyl-1,4-diaminobenzène ou leurs sels physiologiquement acceptables.

4. Composition pour la teinture par oxydation de fibres kératiniques, à base d'une association de coupleur-développeur, **caractérisée en ce qu'**elle contient en tant que développeur au moins un dérivé de N-hétéroarylméthyl-p-phénylènediamine de formule (I) selon l'une quelconque des revendications 1 à 3.

5. Composition selon la revendication 4, **caractérisée en ce qu'**elle contient le composé de formule (I) en une quantité de 0,005 à 20,0 % en poids.

6. Composition selon la revendication 4 ou 5, **caractérisée en ce que** le coupleur est choisi dans le groupe constitué par la 2,6-diaminopyridine, le 2-amino-4-[(2-hydroxyéthyl)amino]anisole, le 2,4-diamino-1-fluoro-5-méthylbenzène, le 2,4-diamino-1-méthoxy-5-méthylbenzène, le 2,4-diamino-1-éthoxy-5-méthylbenzène, le 2,4-diamino-1-(2-hydroxyéthoxy)-5-méthylbenzène, le 2,4-di[(2-hydroxyéthyl)amino]-1,5-diméthoxybenzène, la 2,3-diamino-6-méthoxypyridine, la 3-amino-6-méthoxy-2-(méthylamino)pyridine, la 2,6-diamino-3,5-diméthoxypyridine, la 3,5-diamino-2,6-diméthoxy-pyridine, le 1,3-diaminobenzène, le 2,4-diamino-1-(2-hydroxyéthoxy)benzène, le 2,4-diamino-1,5-di(2-hydroxyéthoxy)benzène, le 1-(2-aminoéthoxy)-2,4-diaminobenzène, le 2-amino-1-(2-hydroxyéthoxy)-4-méthylaminobenzène, l'acide 2,4-diaminophénoxy-acétique, la 3-[di(2-hydroxyéthyl)amino]aniline, le 4-amino-2-di[(2-hydroxyéthyl)amino]-1-éthoxy-benzène, le 5-méthyl-2-(1-méthyléthyl)phénol, la 3-[(2-hydroxyéthyl)amino]aniline, la 3-[(2-amino-éthyl)amino]aniline, le 1,3-di(2,4-diamino-phénoxy)propane, le di(2,4-diaminophénoxy)méthane, le 1,3-diamino-2,4-diméthoxybenzène, le 2,6-bis(2-hydroxyéthyl)aminotoluène, le 4-hydroxyindole, le 3-diméthylaminophénol, le 3-diéthylaminophénol, le 5-amino-2-méthylphénol, le 5-amino-4-fluoro-2-méthylphénol, le 5-amino-4-méthoxy-2-méthylphénol, le 5-amino-4-éthoxy-2-méthylphénol, le 3-amino-2,4-dichlorophénol, le 5-amino-2,4-dichlorophénol, le 3-amino-2-méthylphénol, le 3-amino-2-chloro-6-méthylphénol, le 3-aminophénol, le 2-[(3-hydroxy-phényl)amino]acétamide, le 5-[(2-hydroxyéthyl)-amino]-2-méthylphénol, le 3-[(2-hydroxyéthyl)-amino]phénol, le 3-[(2-méthoxyéthyl)amino]phénol, le 5-amino-2-éthylphénol, le 2-(4-amino-2-hydroxyphénoxy)éthanol, le 5-[(3-hydroxypropyl)-amino]-2-méthylphénol, le 3-[(2,3-dihydroxy-propyl)amino]-2-méthylphénol, le 3-[(2-hydroxy-éthyl)amino]-2-méthylphénol, la 2-amino-3-hydroxy-pyridine, le 5-amino-4-chloro-2-méthylphénol, le 1-naphtol, le 1,5-dihydroxynaphtalène, le 1,7-dihydroxynaphtalène, le 2,3-dihydroxynaphtalène, le 2,7-dihydroxynaphtalène, l'acétate de 2-méthyl-1-naphtol, le 1,3-dihydroxybenzène, le 1-chloro-2,4-dihydroxybenzène, le 2-chloro-1,3-dihydroxy-benzène, le 1,2-dichloro-3,5-dihydroxy-4-méthyl-benzène, le 1,5-dichloro-2,4-dihydroxybenzène, le 1,3-dihydroxy-2-méthylbenzène, le 3,4-méthylène-dioxophénol, la 3,4-méthylènedioxyaniline, le 5-[(2-hydroxyéthyl)amino]-1,3-benzodioxole, le 6-bromo-1-hydroxy-3,4-méthylènedioxybenzène, l'acide 3,4-diaminobenzoïque, la 3,4-dihydro-6-hydroxy-1,4(2H)-benzoxazine, la 6-amino-3,4-dihydro-1,4(2H)-benzoxazine, la 3-méthyl-1-phényl-5-pyrazolone, le 5,6-dihydroxyindole, la 5,6-dihydroxyindoline, le 5-hydroxyindole, le 6-hydroxyindole, le 7-hydroxyindole et la 2,3-indolinedione.

7. Composition selon l'une quelconque des revendications 4 à 6, **caractérisée en ce que** les développeurs et les coupleurs sont contenus chacun, par rapport à la quantité totale de la composition de teinture, en une quantité totale de 0,005 à 20 % en poids.

8. Composition selon l'une quelconque des revendications 4 à 7, **caractérisée en ce qu'**en outre elle contient au moins un colorant direct.

9. Composition selon l'une quelconque des revendications 4 à 8, **caractérisée en ce qu'**il s'agit d'une composition de teinture pour cheveux.
